# EUROPEAN PATENT APPLICATION

(11) **EP 4 818 205 A1**
(43) Date of publication of application: **07.10.2026**
(21) Application number: 24895350.7
(22) Date of filing: 20.03.2024
(51) Int. Cl.: C07K 16/28, C07K 16/24, A61K 39/395, A61P 29/00, A61P 37/00

(54) **ANTIBODY AGAINST TUMOR NECROSIS FACTOR-LIKE LIGAND 1A AND USE THEREOF**

(30) Priority: 30.11.2023 CN 202311632076
(71) Applicant: Beijing Wisdomab Biotechnology Co., Ltd, Beijing 101111 (CN); Genrix(Shanghai) Biopharmaceutical Co., Ltd., Shanghai 201203 (CN); Chongqing Genrix Biopharmaceutical Co., Ltd., Chongqing 401319 (CN)
(72) Inventor: HAO, Xiaobo, Beijing 100163 (CN); LIU, Zhigang, Beijing 100039 (CN); ZHOU, Xiaowei, Beijing 100039 (CN); WAN, Shunan, Beijing 101100 (CN); ZHANG, Suhua, Beijing 102600 (CN); HU, Junjie, Beijing 100176 (CN); LIU, Yulan, Beijing 100176 (CN)
(74) Representative: Schnappauf, Georg
(86) International application number: PCT/CN2024/082756
(87) International publication number: WO 2025/112237

(57) **Abstract**

The present application discloses an antibody binding to a tumor necrosis factor-like ligand 1A (TL1A), a nucleic acid molecule encoding the antibody, a vector comprising the nucleic acid molecule, a host cell comprising the nucleic acid molecule or the vector, a method for preparing and purifying the antibody, and a use of the antibody.

## Description

### CROSS-REFERENCE TO RELATED APPLICATION

The present application claims priority to Chinese Patent Application No. 202311632076.1, filed on November 30, 2023, which is incorporated herein by reference in its entirety.

### TECHNICAL FIELD

The present application pertains to the field of genetic engineering and antibody drugs, and more particularly, relates to an antibody that binds to tumor necrosis factor-like ligand 1A and use of the antibody.

### BACKGROUND

Tumor necrosis factor-like ligand 1A (TL1A/VEGI-251/TNFSF15) is a member of the tumor necrosis factor family, having a full length of 251 amino acids and being a type II transmembrane glycoprotein. As a type II transmembrane protein, like other members of the TNF family, TL1A forms a stable non-covalent homotrimeric structure consisting of β-sandwich folds. TL1A is expressed in immune cells (e.g., monocytes, macrophages, dendritic cells, T lymphocytes, plasma cells, etc.) and non-immune cells (e.g., endothelial cells, synovial fibroblasts, etc). TL1A initially exists in a membrane-bound form and can be cleaved at amino acid position 72 via alternative splicing or tumor necrosis factor-a converting enzyme, thereby being released and functioning as a soluble protein ¹. The functional receptor of TL1A is death receptor 3 (DR3), and the non-functional receptor for TL1A is decoy receptor 3 (DcR3) ². TLIA induces oligomerization of DR3 receptors via binding to DR3, recruits the adaptor protein, TNFR-associated death domain protein (TRADD) via the intracellular death domain, and modulates downstream pathways (e.g., TRAF2, RIP1, PI3K, MAPK, and NF-κB) to exert pro-inflammatory effects; in addition, TL1A promotes apoptosis via the FADD, RIP3, and caspase-8/-3/-7 pathways. Meanwhile, the NF-κB pathway can also activate c-IAP proteins, thereby negatively regulating apoptosis ^{1,3}.

Aberrant expression of TL1A is significantly associated with autoimmune diseases, including rheumatoid arthritis, inflammatory bowel disease, psoriasis, primary biliary cirrhosis, systemic lupus erythematosus, ankylosing spondylitis, etc ¹. The pivotal roles of the TL1A/DR3 signaling pathway in autoimmunity and inflammatory diseases indicate that inhibition of the TL1A-DR3 interaction may serve as an effective therapeutic strategy for alleviating autoimmune diseases and local inflammation in target organs. Clinical indications of currently developed TL1A-targeting monoclonal antibodies include inflammatory bowel disease and systemic sclerosis-associated interstitial lung disease, etc. Among these antibodies, RVT3101 and PRA023 have both demonstrated significant clinical remission rates and endoscopic improvement rates in clinical trials for the treatment of inflammatory bowel disease.

Inflammatory bowel disease (IBD), including Crohn's disease (CD) and ulcerative colitis (UC), is a recurrent inflammatory disease affecting the digestive tract, pathologically characterized by intestinal inflammation and epithelial injury. The etiology of IBD remains incompletely understood and may involve multiple factors such as genetics, environment, intestinal microflora, intestinal immunity, etc.⁴ Current therapeutic drugs for IBD include aminosalicylic acid preparations, oral glucocorticoids, oral small-molecule Janus kinase inhibitors, tumor necrosis factor inhibitors, integrin receptor antagonists, interleukin (IL)-12/23 antagonists, and the like ⁵. Nevertheless, the aforementioned therapeutic regimens still fail to elicit sufficient responses in IBD patients, leaving major clinical needs in the treatment of IBD.

Accordingly, the development and application of novel anti-human TL1A antibodies are highly desirable in the art and possess important biological and medical significance.

### SUMMARY OF THE INVENTION

In a first aspect, the present application provides an antibody that binds to tumor necrosis factor-like ligand 1A (TL1A) comprising a heavy chain variable region and a light chain variable region.

In some embodiments of the first aspect, the heavy chain variable region comprises amino acid sequences of HCDR1, HCDR2 and HCDR3 as set forth in SEQ ID NO: 1, 2 and 3, and the light chain variable region comprises the amino acid sequences of LCDR1, LCDR2 and LCDR3 as set forth in SEQ ID NOs: 4, 5 and 6.

In some embodiments of the first aspect, the heavy chain variable region comprises amino acid sequences of HCDR1, HCDR2 and HCDR3 as set forth in SEQ ID NO: 7, 8 and 9, and the light chain variable region comprises the amino acid sequences of LCDR1, LCDR2 and LCDR3 as set forth in SEQ ID NO: 10, 11 and 12.

In some embodiments of the first aspect, the heavy chain variable region comprises amino acid sequences of HCDR1, HCDR2 and HCDR3 as set forth in SEQ ID NO: 7, 13 and 9, and the light chain variable region comprises the amino acid sequences of LCDR1, LCDR2 and LCDR3 as set forth in SEQ ID NO: 14, 15 and 16.

In some embodiments of the first aspect, the heavy chain variable region comprises amino acid sequences of HCDR1, HCDR2 and HCDR3 as set forth in SEQ ID NO: 17, 18 and 19, and the light chain variable region comprises the amino acid sequences of LCDR1, LCDR2 and LCDR3 as set forth in SEQ ID NO: 20, 21 and 22.

The amino acid sequences of HCDRs and LCDRs are defined according to Kabat numbering scheme.

In some embodiments of the first aspect, the amino acid sequence of the heavy chain variable region of the antibody is as set forth in SEQ ID NO: 23, 24, 25, or 26.

In some embodiments of the first aspect, the amino acid sequence of the light chain variable region of the antibody is as set forth in SEQ ID NO: 27, 28, 29, or 30.

In some embodiments of the first aspect, the amino acid sequence of the heavy chain variable region of the antibody is as set forth in SEQ ID NO: 23, and the amino acid sequence of the light chain variable region of the antibody is as set forth in SEQ ID NO: 27.

In some embodiments of the first aspect, the amino acid sequence of the heavy chain variable region of the antibody is as shown in SEQ ID NO: 24, and the amino acid sequence of the light chain variable region of the antibody is as shown in SEQ ID NO: 28.

In some embodiments of the first aspect, the amino acid sequence of the heavy chain variable region of the antibody is as set forth in SEQ ID NO: 25, and the amino acid sequence of the light chain variable region of the antibody is as set forth in SEQ ID NO: 29.

In some embodiments of the first aspect, the amino acid sequence of the heavy chain variable region of the antibody is as set forth in SEQ ID NO: 26, and the amino acid sequence of the light chain variable region of the antibody is as set forth in SEQ ID NO: 30.

In some embodiments of the first aspect, the amino acid sequence of the heavy chain variable region of the antibody has at least 90% identity to SEQ ID NO: 23, 24, 25, or 26, and the amino acid sequence of the light chain variable region of the antibody has at least 90% identity to SEQ ID NO: 27, 28, 29, or 30.

In some embodiments of the first aspect, the antibody is an intact antibody, a Fab fragment, an F(ab')₂ fragment, or a single-chain Fv fragment (scFv).

In some embodiments of the first aspect, the antibody is a monoclonal antibody.

In some embodiments of the first aspect, the antibody further comprises a heavy chain constant region selected from the group consisting of an IgG1 subtype, an IgG2 subtype, and an IgG4 subtype.

In some embodiments of the first aspect, the antibody further comprises a light chain constant region selected from the group consisting of a κ subtype and a λ subtype.

In some embodiments of the first aspect, the antibody binds to TL1A of a primate and/or a rodent.

In some embodiments of the first aspect, the antibody inhibits binding of TL1A to death receptor 3 (DR3).

In some embodiments of the first aspect, the antibody does not inhibit binding of TL1A to decoy receptor 3 (DcR3).

In some embodiments of the first aspect, the antibody inhibits caspase activation in TL1A-stimulated cells; and/or

In some embodiments of the first aspect, the antibody inhibits NF-κB activation in TL1A-stimulated cells.

In some embodiments of the first aspect, the antibody inhibits the ability of TL1A-stimulated PBMCs to secrete interferon-γ.

In a second aspect, the present application provides a nucleic acid molecule encoding the antibody of the first aspect.

In a third aspect, the present application provides a pharmaceutical composition comprising the antibody of the first aspect and a pharmaceutically acceptable excipient, diluent or carrier.

In a fourth aspect, the present application provides use of the antibody of the first aspect, or the pharmaceutical composition of the third aspect in the manufacture of a medicament for the prevention or treatment of a TL1A-related disease.

In a fifth aspect, the present application provides a method for preventing or treating a TL1A-related disease, comprising administering to a subject in need thereof the antibody of the first aspect, or the pharmaceutical composition of the third aspect.

### BRIEF DESCRIPTION OF THE DRAWINGS

FIG. 1 shows the results demonstrating that the mouse anti-TL1A monoclonal antibodies inhibit caspase activation in TL1A-stimulated TF-1 cells.
FIG. 2 shows the results demonstrating that the mouse anti-TL1A monoclonal antibodies inhibit NF-κB activation in TL1A-stimulated 293T-hDR3-NF-κB-RE-luci cells.
FIG. 3 shows the results demonstrating that the anti-TL1A monoclonal antibody H3F1+L28E1 inhibits caspase activation in TL1A-stimulated TF-1 cells.
FIG. 4 shows the results demonstrating that the anti-TL1A monoclonal antibody H3F1+L28E1 inhibits NF-κB activation in TL1A-stimulated 293T-hDR3-NF-κB-RE-luci cells.
FIG. 5 shows the ability of the anti-TL1A monoclonal antibody H3F1+L28E1 to inhibit TL1A-stimulated PBMCs to secrete interferon-γ.
FIG. 6 shows the ELISA analysis results demonstrating that the anti-human TL1A monoclonal antibodies block the binding of human TL1A to human DR3.
FIG. 7 shows ELISA analysis results demonstrating that the anti-TL1A monoclonal antibodies block the binding activity of human DcR3 to human TL1A.
FIG. 8 shows the results of fecal scores in DNBS-induced rat acute ulcerative colitis.
FIG. 9 shows the results of colonic macroscopic injury scores in DNBS-induced rat acute ulcerative colitis.

### DESCRIPTION OF SEQUENCE

SEQ ID NO:1-3 show the amino acid sequences of HCDR1, HCDR2 and HCDR3 of the anti-TL1A single-chain antibody S4D3, respectively.
SEQ ID NO:4-6 show the amino acid sequences of LCDR1, LCDR2 and LCDR3 of the anti-TL1A single-chain antibody S4D3, respectively.
SEQ ID NO:7 shows the amino acid sequence of HCDR1 of the anti-TL1A single-chain antibodies S9H9 and S7B11.
SEQ ID NO:8 shows the amino acid sequence of HCDR2 of the anti-TL1A single-chain antibody S9H9.
SEQ ID NO:9 shows the amino acid sequence of HCDR3 of the anti-TL1A single-chain antibodies S9H9 and S7B11.
SEQ ID NO:10-12 show the amino acid sequences of LCDR1, LCDR2 and LCDR3 of the anti-TL1A single-chain antibody S9H9, respectively.
SEQ ID NO:13 shows the amino acid sequence of HCDR2 of the anti-TL1A single-chain antibody S7B11.
SEQ ID NO:14-16 show the amino acid sequences of LCDR1, LCDR2 and LCDR3 of the anti-TL1A single-chain antibody S7B11, respectively.
SEQ ID NO:17-19 show the amino acid sequences of HCDR1, HCDR2 and HCDR3 of the fully-humanized anti-TL1A monoclonal antibody H3F1+L28E1, respectively.
SEQ ID NO:20-22 show the amino acid sequences of LCDR1, LCDR2 and LCDR3 of the fully-humanized anti-TL1A monoclonal antibody H3F1+L28E1, respectively.
SEQ ID NO:23 shows the amino acid sequence of the heavy chain variable region of the anti-TL1A single-chain antibody S4D3.
SEQ ID NO:24 shows the amino acid sequence of the heavy chain variable region of the anti-TL1A single-chain antibody S9H9.
SEQ ID NO:25 shows the amino acid sequence of the heavy chain variable region of the anti-TL1A single-chain antibody S7B11.
SEQ ID NO:26 shows the amino acid sequence of the heavy chain variable region of the fully-humanized anti-TL1A monoclonal antibody H3F1+L28E1.
SEQ ID NO:27 shows the amino acid sequence of the light chain variable region of the anti-TL1A single-chain antibody S4D3.
SEQ ID NO:28 shows the amino acid sequence of the light chain variable region of the anti-TL1A single-chain antibody S9H9.
SEQ ID NO:29 shows the amino acid sequence of the light chain variable region of the anti-TL1A single-chain antibody S7B11.
SEQ ID NO:30 shows the amino acid sequence of the light chain variable region of the fully-humanized anti-TL1A monoclonal antibody H3F1+L28E1.
SEQ ID NO:31 shows the amino acid sequence of the recombinant human TL1A (hTL1A).
SEQ ID NO:32 shows the amino acid sequence of the recombinant *Macaca fascicularis* TL1A (mfTL1A).
SEQ ID NO:33 shows the amino acid sequence of the recombinant mouse TL1A (mTL1A).
SEQ ID NO:34 shows the amino acid sequence of the recombinant rat TL1A (rTL1A).
SEQ ID NO:35 shows the amino acid sequence of the Foldon domain (Fib Foldon) of T4 phage fibrin.
SEQ ID NO:36 shows the amino acid sequence of the His tag.
SEQ ID NO:37 shows the amino acid sequence of the human (*Homo sapiens*) IgG1 subtype heavy chain constant region (CH-IgG1).
SEQ ID NO:38 shows the amino acid sequence of the human IgG1 subtype heavy chain constant region mutant IgG1m3 (CH-IgG1m3).
SEQ ID NO:39 shows the amino acid sequence of the human IgG1 subtype heavy chain constant region mutant IgG1m3-YTE (CH-IgG1m3-YTE).
SEQ ID NO:40 shows the amino acid sequence of the human (*Homo sapiens*) κ-isotype light chain constant region.
SEQ ID NO:41 shows the amino acid sequence of the human (*Homo sapiens*) λ-isotype light chain constant region.
SEQ ID NO:42 shows the amino acid sequence of the recombinant protein His-fib-hTL1A.
SEQ ID NO:43 shows the amino acid sequence of the recombinant protein His-fib-mfTL1A.
SEQ ID NO:44 shows the amino acid sequence of the recombinant protein His-fib-mTL1A.
SEQ ID NO:45 shows the amino acid sequence of the recombinant protein His-fib-rTL1A.
SEQ ID NO:46 shows the nucleotide sequence of the primer PmCGR.
SEQ ID NO:47 shows the nucleotide sequence of the primer PmCKR.
SEQ ID NO:48 shows the amino acid sequence of the single-chain antibody S4D3.
SEQ ID NO:49 shows the amino acid sequence of the single-chain antibody S9H9.
SEQ ID NO:50 shows the amino acid sequence of the single-chain antibody S7B11.
SEQ ID NO:51 shows the amino acid sequence of the heavy chain variable region of the anti-TL1A monoclonal antibody RVT-3101.
SEQ ID NO:52 shows the amino acid sequence of the light chain variable region of the anti-TL1A monoclonal antibody RVT-3101.
SEQ ID NO:53 shows the amino acid sequence of the heavy chain variable region of the anti-TL1A monoclonal antibody PRA023.
SEQ ID NO:54 shows the amino acid sequence of the light chain variable region of the anti-TL1A monoclonal antibody PRA023.
SEQ ID NO:55 shows the amino acid sequence of the heavy chain variable region of the negative control antibody DP47.
SEQ ID NO:56 shows the amino acid sequence of the light chain variable region of the negative control antibody DP47.

### DETAILED DESCRIPTION OF THE INVENTION

The inventors of the present application have obtained a new antibody that binds to tumor necrosis factor-like ligand 1A (TL1A) through antibody engineering techniques. In various aspects of the present application, provided are a new anti-tumor necrosis factor-like ligand 1A (TL1A) antibody, a nucleic acid molecule encoding the antibody, a vector comprising the nucleic acid molecule, a host cell comprising the nucleic acid molecule or vector, a method of preparing and purifying the antibody, and medical and biological uses of the antibody. A full-length antibody molecule can be constructed as a medicament for preventing or treating TL1A-related diseases according to the amino acid sequences of the variable regions of the antibody provided herein.

Unless otherwise indicated, the practice of the present application employs conventional molecular biology, microbiology, cell biology, biochemistry, and immunological techniques in the art.

Unless otherwise indicated, the terms used in the present application have the meanings commonly understood by those skilled in the art.

### Definition

As used herein, the term "antibody" refers to an immunoglobulin molecule capable of specifically binding to a target via at least one antigen recognition site located in its variable region. Targets include, but are not limited to, carbohydrates, polynucleotides, lipids, polypeptides, and the like. As used herein, "antibody" includes not only an intact (*i.e.*, full-length) antibody, but also a binding fragment thereof (e.g., Fab, Fab', F(ab')₂, or Fv), a variant thereof, a fusion protein comprising an antibody moiety, a humanized antibody, a chimeric antibody, a diabody, a linear antibody, a single-chain antibody, a multi-specific antibody (e.g., a bispecific antibody), and any other modified configurations of an immunoglobulin molecule comprising a desired specific antigen recognition site, including a glycosylated variant of an antibody, an amino acid sequence variant of an antibody, and a covalently modified antibody.

Typically, an intact or full-length antibody comprises two heavy chains and two light chains. Each heavy chain contains the heavy chain variable region (VH) and the first, second and third constant regions (CH1, CH2 and CH3). Each light chain contains the light chain variable region (VL) and the constant region (CL). A full-length antibody may belong to any immunoglobulin class, such as IgD, IgE, IgG, IgA, or IgM (or any of the above subclasses), but it is not necessarily restricted to any particular class. Immunoglobulins can be categorized based on the amino acid sequences of their heavy chain constant regions. Generally, there are five primary classes, *i.e.*, IgA, IgD, IgE, IgG, and IgM, some of which can be further classified into subclasses (subtypes), such as IgG1, IgG2, IgG3, IgG4, IgA1, and IgA2. The heavy chain constant regions corresponding to these individual immunoglobulin classes are referred to as α, δ, ε, γ, and µ, respectively. Subunit structures and three-dimensional conformations of different types of immunoglobulins are well characterized.

As used herein, the term "antigen-binding fragment" or "antigen-binding moiety" refers to the portion or region of an intact antibody molecule responsible for binding to an antigen. The antigen- binding domain may comprise a heavy chain variable region (VH), a light chain variable region (VL), or both. Each of the VH and VL region typically contains three complementarity-determining regions, *i.e.*, CDR1, CDR2, and CDR3.

It is well known to those skilled in the art that complementarity-determining regions (CDRs, typically including CDR1, CDR2 and CDR3) are the portions of a variable region having the greatest impact on the affinity and specificity of the antibody. The CDR sequence of the VH or VL has two common schemes, *i.e.*, the Chothia numbering scheme and the Kabat numbering scheme. See, e.g., Kabat, "Sequences of Proteins of Immunological Interest", National Institutes of Health, Bethesda, Md. (1991) ⁷; A1-Lazikani et al., J. Mol. Biol. 273:927-948 (1997) ⁸; and Martin et al., Proc. Natl. Acad. Sci. USA. 86: 9268-9272 (1989) ⁹. For a given antibody variable region amino acid sequence, the CDR amino acid sequences in the VH and VL amino acid sequences can be determined according to the Chothia or Kabat numbering scheme. In an embodiment of the present application, the CDR amino acid sequences are defined according to the Kabat numbering scheme.

For a given antibody variable region amino acid sequence, the CDR amino acid sequences in the variable region can be determined in various ways, for example, using the online software Abysis (http://www.abysis.org/).

Examples of an antigen-binding fragment include, but are not limited to: (1) a Fab fragment, which may be a monovalent fragment consisting of a VL-CL chain and a VH-CH1 chain; (2) an F(ab')₂ fragment, which may be a divalent fragment consisting of two Fab' fragments linked via a disulfide bridge of the hinge region (*i.e.*, a dimer of Fab'); (3) an Fv fragment consisting of VL and VH domains from a single arm of an antibody; (4) a single-chain Fv (scFv), which may be a single polypeptide chain consisting of a VH domain and a VL domain via a peptide linker; and (5) (scFv)₂, which may comprise two VH domains linked via a peptide linker and two VL domains that associate with the two VH domains via disulfide bridges.

The term "specific binding" as used herein refers to a non-random binding reaction between two molecules, e.g., the binding of an antibody to an antigen epitope.

As used herein, the term "monoclonal antibody" refers to an antibody derived from a substantially homogeneous population of antibodies, *i.e.*, each antibody of the population is identical, aside from rare naturally occurring mutations.

In a first aspect, the present application provides an antibody that binds to tumor necrosis factor-like ligand 1A (TL1A) comprising a heavy chain variable region and a light chain variable region.

In some embodiments of the first aspect, the heavy chain variable region of the antibody comprises the amino acid sequences of HCDR1, HCDR2 and HCDR3 as set forth in SEQ ID NO: 1, 2 and 3, and the light chain variable region of the antibody comprises the amino acid sequences of LCDR1, LCDR2 and LCDR3 as set forth in SEQ ID NO: 4, 5 and 6.

In some embodiments of the first aspect, the heavy chain variable region of the antibody comprises the amino acid sequences of HCDR1, HCDR2 and HCDR3 as set forth in SEQ ID NO: 7, 8 and 9, and the light chain variable region of the antibody comprises the amino acid sequences of LCDR1, LCDR2 and LCDR3 as set forth in SEQ ID NO: 10, 11 and 12.

In some embodiments of the first aspect, the heavy chain variable region of the antibody comprises the amino acid sequences of HCDR1, HCDR2 and HCDR3 as set forth in SEQ ID NO: 7, 13 and 9, and the light chain variable region of the antibody comprises the amino acid sequences of LCDR1, LCDR2 and LCDR3 as set forth in SEQ ID NO: 14, 15 and 16.

In some embodiments of the first aspect, the heavy chain variable region of the antibody comprises the amino acid sequences of HCDR1, HCDR2 and HCDR3 as set forth in SEQ ID NO: 17, 18 and 19, and the light chain variable region of the antibody comprises the amino acid sequences of LCDR1, LCDR2 and LCDR3 as set forth in SEQ ID NO: 20, 21 and 22.

In the above embodiments, the amino acid sequences of HCDRs and LCDRs are defined according to Kabat numbering scheme.

In some embodiments of the first aspect, the amino acid sequence of the heavy chain variable region of the antibody is as set forth in SEQ ID NO: 23, 24, 25, or 26.

In some embodiments of the first aspect, the amino acid sequence of the light chain variable region of the antibody is as set forth in SEQ ID NO: 27, 28, 29, or 30.

In some embodiments of the first aspect, the amino acid sequence of the heavy chain variable region of the antibody is as set forth in SEQ ID NO: 23, and the amino acid sequence of the light chain variable region of the antibody is as set forth in SEQ ID NO: 27.

In some embodiments of the first aspect, the amino acid sequence of the heavy chain variable region of the antibody is as set forth in SEQ ID NO: 24, and the amino acid sequence of the light chain variable region of the antibody is as set forth in SEQ ID NO: 28.

In some embodiments of the first aspect, the amino acid sequence of the heavy chain variable region of the antibody is as set forth in SEQ ID NO: 25, and the amino acid sequence of the light chain variable region of the antibody is as set forth in SEQ ID NO: 29.

In some embodiments of the first aspect, the amino acid sequence of the heavy chain variable region of the antibody is as set forth in SEQ ID NO: 26, and the amino acid sequence of the light chain variable region of the antibody is as set forth in SEQ ID NO: 30.

In some embodiments of the first aspect, the amino acid sequence of the heavy chain variable region of the antibody has at least 90% identity to SEQ ID NO: 23, 24, 25, or 26, and the amino acid sequence of the light chain variable region of the antibody has at least 90% identity to SEQ ID NO: 27, 28, 29, or 30.

In some embodiments of the first aspect, the amino acid sequence of the heavy chain variable region of the antibody has at least 90%, 91%, 92%, 93%, 94%, 95%, 96%, 97%, 98%, 99%, or higher identity to SEQ ID NO: 23, 24, 25, or 26.

In some embodiments of the first aspect, the amino acid sequence of the light chain variable region of the antibody has at least 90%, 91%, 92%, 93%, 94%, 95%, 96%, 97%, 98%, 99%, or higher identity to SEQ ID NO: 27, 28, 29, or 30.

In some embodiments of the first aspect, the amino acid sequence of the heavy chain variable region of the antibody differs from the amino acid sequence set forth in SEQ ID NO: 23, 24, 25, or 26 by about 1, 2, 3, 4, 5, 6, 7, 8, 9, or 10 amino acid substitutions, deletions, and/or additions.

In some embodiments of the first aspect, the amino acid sequence of the light chain variable region of the antibody differs from the amino acid sequence set forth in SEQ ID NO: 27, 28, 29, or 30 by about 1, 2, 3, 4, 5, 6, 7, 8, 9, or 10 amino acid substitutions, deletions, and/or additions.

In some embodiments of the first aspect, the amino acid sequence as set forth in SEQ ID NO: 23, 24, 25, or 26 may also be truncated by about 1, 2, 3, 4, 5, 6, 7, 8, 9, 10, 11, 12, 13, 14, 15, 20, 25, or more amino acids at the C-terminus or N-terminus, while still retaining a function similar to that of the antibody heavy chain variable region.

In some embodiments of the first aspect, the amino acid sequence as set forth in SEQ ID NO: 23, 24, 25 or 26 may also have 1, 2, 3, 4, 5, 6, 7, 8, 9, 10, 11, 12, 13, 14, 15, 20, 25 or more amino acids added at the C-terminus or N-terminus, while still retaining a function similar to that of the antibody heavy chain variable region.

In some embodiments of the first aspect, the amino acid sequence as set forth in SEQ ID NO: 23, 24, 25, or 26 may also have 1, 2, 3, 4, 5, 6, 7, 8, 9, 10, 11, 12, 13, 14, 15, 20, 25 or more amino acids added or deleted at regions other than the C-terminus or N-terminus, provided that the altered amino acid sequence substantially retains a function similar to that of the antibody heavy chain variable region.

In some embodiments of the first aspect, the amino acid sequence as set forth in SEQ ID NO: 27, 28, 29, or 30 may also be truncated by about 1, 2, 3, 4, 5, 6, 7, 8, 9, 10, 11, 12, 13, 14, 15, 20, 25, or more amino acids at the C-terminus or N-terminus, while still retaining a function similar to that of the antibody light chain variable region.

In some embodiments of the first aspect, the amino acid sequence as set forth in SEQ ID NO: 27, 28, 29, or 30 may also have 1, 2, 3, 4, 5, 6, 7, 8, 9, 10, 11, 12, 13, 14, 15, 20, 25 or more amino acids added at the C-terminus or N-terminus, while still retaining a function similar to that of the antibody light chain variable region.

In some embodiments of the first aspect, the amino acid sequence as set forth in SEQ ID NO: 27, 28, 29, or 30 may also have 1, 2, 3, 4, 5, 6, 7, 8, 9, 10, 11, 12, 13, 14, 15, 20, 25 or more amino acids added or deleted at regions other than the C-terminus or N-terminus, provided that the altered amino acid sequence substantially retains a function similar to that of the antibody light chain variable region.

In some embodiments of the first aspect, the antibody is an intact antibody, a Fab fragment, an F(ab')₂ fragment, or a single-chain Fv fragment (scFv).

In some embodiments of the first aspect, the antibody is a fully-humanized antibody.

In some embodiments of the first aspect, the antibody is a monoclonal antibody.

In some embodiments of the first aspect, the antibody further comprises a heavy chain constant region selected from the group consisting of an IgG1 subtype, an IgG2 subtype, and an IgG4 subtype.

In some embodiments of the first aspect, the heavy chain constant region is of IgG1 subtype.

In some embodiments of the first aspect, the heavy chain constant region comprises an Fc fragment of an IgG1 subtype heavy chain constant region, and the Fc fragment comprises the amino acid F at position 234, the amino acid E at position 235, and the amino acid S at position 331; wherein the amino acid positions of the antibody constant region are determined according to the EU numbering scheme.

In some embodiments of the first aspect, the heavy chain constant region comprises an Fc fragment of an IgG1 subtype heavy chain constant region, and the Fc fragment comprises the amino acid Y at position 252, the amino acid T at position 254, and the amino acid E at position 256; wherein the amino acid positions of the antibody constant region are determined according to the EU numbering scheme.

In some embodiments of the first aspect, the heavy chain constant region comprises an Fc fragment sequence of an IgG1 subtype heavy chain constant region, and the Fc fragment comprises the amino acid F at position 234, the amino acid E at position 235, and the amino acid S at position 331, and the Fc fragment comprises the amino acid Y at position 252, the amino acid T at position 254, and the amino acid E at position 256; wherein the amino acid positions of the antibody constant region are determined according to the EU numbering scheme.

In some embodiments of the first aspect, the antibody further comprises a light chain constant region selected from the group consisting of κ isotype and λ isotype.

In some embodiments of the first aspect, the antibody binds to TL1A of a primate. In some embodiments, the primate is a human, or a member of the Cercopithecidae, such as *Macaca fascicularis*.

In some embodiments of the first aspect, the antibody binds to TL1A of a rodent. In some embodiments, the rodent is a member of the Muridae, such as a rat or a mouse.

In some embodiments of the first aspect, the antibody binds to the recombinant human TL1A (SEQ ID NO: 31).

In some embodiments of the first aspect, the antibody binds to the recombinant monkey (e.g., *Macaca fascicularis*) TL1A (SEQ ID NO: 32).

In some embodiments of the first aspect, the antibody binds to the recombinant mouse TL1A (SEQ ID NO:33).

In some embodiments of the first aspect, the antibody binds to the recombinant rat TL1A (SEQ ID NO: 34).

In some embodiments of the first aspect, the antibody inhibits binding of TL1A to death receptor 3 (DR3).

In some embodiments of the first aspect, the antibody does not inhibit binding of TL1A to decoy receptor 3 (DcR3).

In some embodiments of the first aspect, the antibody inhibits caspase activation in TL1A-stimulated cells (e.g., tumor cells, e.g., TF-1 cells).

In some embodiments of the first aspect, the antibody inhibits NF-κB activation in TL1A-stimulated cells (e.g., 293T-hDR3-NF-κB-RE-luci cells).

In some embodiments of the first aspect, the antibody inhibits the ability of TL1A-stimulated PBMCs to secrete interferon-γ.

In a second aspect, the present application provides a nucleic acid molecule encoding the antibody of the first aspect.

In some embodiments, the nucleic acid molecule is operably linked to a regulatory nucleotide sequence that can be recognized by a host cell transformed with a vector.

In a third aspect, the present application provides a pharmaceutical composition comprising the antibody of the first aspect and a pharmaceutically acceptable excipient, diluent or carrier.

In some embodiments of the third aspect, the pharmaceutical composition is used to prevent or treat a TL1A-related disease.

In some embodiments of the third aspect, the TL1A-related disease is rheumatoid arthritis, inflammatory bowel disease (e.g., Crohn's disease (CD) and ulcerative colitis (UC)), psoriasis, primary biliary cirrhosis, systemic lupus erythematosus, ankylosing spondylitis, or systemic sclerosis-related interstitial lung disease.

In some embodiments of the third aspect, the pharmaceutical composition may further comprise one or more of: a lubricant, such as talc, magnesium stearate and mineral oil; a wetting agent; an emulsifier; a suspending agent; a preservative, such as benzoic acid, sorbic acid or calcium propionate; a sweetening agent and/or a flavoring agent, and the like.

In some embodiments of the third aspect, the pharmaceutical composition of the present application may be formulated in the form of tablet, pill, powder, lozenge, elixir, suspension, emulsion, solution, syrup, suppository, or capsule, etc.

In some embodiments of the third aspect, the pharmaceutical composition of the present application may be delivered using any physiologically acceptable route of administration including, but not limited to, oral administration, parenteral administration, nasal administration, rectal administration, intraperitoneal administration, intravascular injection, subcutaneous administration, transdermal administration, inhalation, and the like.

In some embodiments of the third aspect, the pharmaceutical composition for therapeutic use can be formulated for storage in the form of a lyophilized formulation or an aqueous solution by mixing reagents having the desired purity with pharmaceutically acceptable carriers, excipients, and the like, as appropriate.

In a fourth aspect, the present application provides use of the antibody of the first aspect and the pharmaceutical composition of the third aspect in the manufacture of a medicament for the prevention or treatment of a TL1A-related disease.

In some embodiments of the fourth aspect, the TL1A-related disease is rheumatoid arthritis, inflammatory bowel disease (e.g., Crohn's disease (CD) and ulcerative colitis (UC)), psoriasis, primary biliary cirrhosis, systemic lupus erythematosus, ankylosing spondylitis, or systemic sclerosis-related interstitial lung disease.

In a fifth aspect, the present application provides a method of preventing or treating a TL1A-related disease comprising administering to a subject in need thereof the antibody of the first aspect, or the pharmaceutical composition of the third aspect.

In some embodiments of the fifth aspect, the TL1A-related disease is rheumatoid arthritis, inflammatory bowel disease (e.g., Crohn's disease (CD) and ulcerative colitis (UC)), psoriasis, primary biliary cirrhosis, systemic lupus erythematosus, ankylosing spondylitis, or systemic sclerosis-related interstitial lung disease.

In other aspects, the present application also provides a vector comprising the nucleic acid molecule encoding the antibody of the present invention or a light or heavy chain thereof, a host cell comprising the nucleic acid molecule or the vector, and a method of producing the antibody. In some embodiments, the nucleic acid molecule is operably linked to a regulatory nucleotide sequence that can be recognized by a host cell transformed with the vector. In some embodiments, the method of producing the antibody includes culturing the host cell to express the nucleic acid. In some embodiments, the method of producing the antibody further comprises recovering the antibody from the host cell culture medium.

It is to be understood that the foregoing detailed description is intended only to enable those skilled in the art to have a clearer understanding of the present application and is not intended to be limiting in any way. Various modifications and variations can be made to the embodiments by those skilled in the art.

### Examples

The following examples are for purposes of illustration only and are not intended to limit the scope of the present application.

### Example 1: Preparation of Recombinant Proteins

A variety of different recombinant proteins, including human TL1A (hTL1A, SEQ ID NO: 31), *Macaca fascicularis* TL1A (mfTL1A, SEQ ID NO: 32), mouse TL1A (mTL1A, SEQ ID NO: 33), and rat TL1A (rTL1A, SEQ ID NO: 34), were utilized for the preparation and identification of anti-TL1A antibodies. The Foldon domain of T4 phage fibritin (Fib Foldon, SEQ ID NO: 35) was added to the N-terminus of these recombinant proteins to maintain the natural trimeric conformation of TL1A and enhance stability. Meanwhile, a His tag (His, SEQ ID NO: 36) was added to the N-terminus of these recombinant proteins to facilitate purification and functional identification of the recombinant proteins. In the preparation of recombinant antibodies, the antibody heavy chain constant region may be the human IgG1 subtype (CH-IgG1, SEQ ID NO: 37) or its various mutants, such as IgG1m3 (CH-IgG1m3, SEQ ID NO: 38) or IgG1m3-YTE (CH-IgG1m3-YTE, SEQ ID NO: 39); and the antibody light chain constant region may be the human kappa (κ) isotype (CK, SEQ ID NO: 40) or human lambda (λ) isotype (CL, SEQ ID NO: 41).

Genes for the above-mentioned various recombinant proteins were designed and synthesized according to the amino acid sequences of the recombinant proteins in the UniProt database. The synthesized genes for the various recombinant proteins were cloned into suitable eukaryotic expression vectors (such as pcDNA3.1, Invitrogen) using conventional techniques in molecular biology. Plasmids prepared for recombinant protein expression were then transfected into HEK293 cells (such as HEK293F, Invitrogen) using liposomes (such as 293fectin, Invitrogen) or other cationic transfection reagents (such as PEI). The cells were cultured in suspension under serum-free conditions for 3-4 days, and the culture supernatant was harvested by centrifugation or the like.

The recombinant proteins expressed as fusions with His tag, i.e., His-fib-hTL1A (SEQ ID NO: 42), His-fib-mfTL1A (SEQ ID NO: 43), His-fib-mTL1A (SEQ ID NO: 44), and His-fib-rTL1A (SEQ ID NO: 45), were subjected to one-step purification from the culture supernatant using a metal chelate affinity chromatography column (e.g., HisTrap FF, GE Inc.). The recombinant antibodies were subjected to one-step purification using a ProteinA/G affinity chromatography column (e.g., MabSelect Sure, GE Inc.). The preservation buffer for the recombinant protein was then replaced with PBS (pH 7.0) or another suitable buffer using a desalting column (e.g., HiTrap Desalting, GE Inc.). The recombinant proteins were sterilized by filtration, and then stored in aliquots at -20°C for future use.

### Example 2: Preparation of Immune Library and Screening

### 2.1 Mouse Immunization and Preparation of Antibody Library

Blood from BALB/c mice aged 6-8 weeks was collected via tail vein to obtain baseline serum before immunization. For the first immunization, His-fib-hTL1A fusion protein was emulsified with Freund's Complete Adjuvant and each mouse was injected with 50 µg of the fusion protein. For booster immunizations every two weeks, His-fib-hTL1A fusion protein was emulsified with Freund's Incomplete Adjuvant, and each mouse was injected with 50 µg of the fusion protein after blood was taken via tail snip for the first and third booster immunizations, and His-fib-mfTL1A was emulsified with Freund's Incomplete Adjuvant and each mouse was injected with 100 µg of the fusion protein for the second and fourth booster immunizations. For the sixth immunization, His-fib-hTL1A recombinant antigen without adjuvant was used as immunogen, and each mouse was injected with 50 µg of the fusion protein. The mice were sacrificed 3 days after high-dose booster immunization and spleen cells were collected.

Mouse spleen lymphocytes were isolated using Mouse Lymphocyte Isolate Solution (Beijing Dakewe Biotech Co., Ltd., CAT#DKW33-R0100). Total RNA was extracted from the lymphocytes using Cell Total RNA Extraction Kit (TIANGEN Biotech (BEIJING) Co., Ltd., CAT#DP430). The extracted total RNA was used as a template to synthesize cDNA for the heavy chain variable region and the light chain variable region using First- Strand cDNA Synthesis Kit (Thermo scientific, CAT#K1621). Gene-specific primers were used as the reverse transcription primers. and the primer binding regions were located in the antibody heavy chain constant region and the antibody light chain constant region, respectively. The specific sequences were PmCGR: TGCATTTGAACTCCTTGCC (SEQ ID NO: 46) and PmCKR: CCATCAATCTTCCACTTGAC (SEQ ID NO: 47). The synthesized cDNAs were immediately stored at -70 °C for future use. The cDNA obtained by reverse transcription was then used as a template to synthesize primers according to the Reference ⁶, and the mouse antibody VH and Vκ genes were amplified by PCR. The single-chain variable fragments (scFv) gene was then constructed using Overlap Extension PCR. Finally, the prepared mouse single-chain antibody gene was cloned into the vector pADSCFV-S (see Chinese Patent Application No. 201510097117.0 ⁷) to construct a scFv library. The antibody library had a capacity of 6.0E+8, with an accuracy of 70%.

### 2.2 Screening of Mouse Single-Chain Antibody Library

The phage library displaying mouse single-chain antibodies constructed in Section 2.1 was screened by a solid phase screening strategy (for the experimental protocol, see Phage Display: A Practical Approach, Clackson, T. (USA) and Lowman, H. B. (USA) (ed.), translated by Lan Ma et al., Chemical Industry Press, May 2008 8) using the His-fib-hTL1A and His-fib-mfTL1A prepared in Example 1 as antigens. Three rounds of screening were performed by means of binding, elution, neutralization, infection and amplification. Finally, three single-chain antibodies of different sequences, i.e., S4D3 (SEQ ID NO: 48), S9H9 (SEQ ID NO: 49), and S7B11 (SEQ ID NO: 50), were obtained.

The single-chain antibodies S4D3, S9H9 and S7B11 were prepared as IgG1m3 allotype intact antibodies using conventional molecular biological methods. Also, the heavy chain variable region (SEQ ID NO: 51) and light chain variable region (SEQ ID NO: 52) of RVT-3103 were synthesized with reference to U.S. Patent Application No. US20150132311A1 ⁹; the heavy chain variable region (SEQ ID NO: 53) and light chain variable region (SEQ ID NO: 54) of PRA23 were synthesized with reference to U.S. Patent Application No. US20210122828A1 ¹⁰. RVT-3101 and PRA023 prepared as IgG1m3 subtype intact antibodies were used as positive controls. DP47 (germline gene antibody, see U.S. Patent Application No. US20160200833A1 ¹¹, DP47VH, SEQ ID NO: 55; DP47Vκ, SEQ ID NO: 56) prepared as an IgG1m3 allotype intact antibody was used as a negative control.

### Example 3: Identification of Mouse Anti-TL1A Monoclonal Antibodies

### 3.1 Affinity Analysis of Mouse Anti-TL1A Monoclonal Antibodies S4D3, S9H9 and S7B11

The affinity of mouse anti-human TL1A monoclonal antibodies was determined by surface plasmon resonance technique (SPR) using Biacore T200. Related reagents and consumables, such as Amine Coupling Kit (BR-1000-50), Human Antibody Capture Kit (BR-1008-39), Series S CM5 Chip (14100530), and 10×HBS-EP buffer (pH 7.4, BR100669), were purchased from GE Healthcare. According to the kit instructions, the carboxylated CM5 Chip surface was activated with 1-Ethyl-3-(3-dimethylaminopropyl) carbodiimide hydrochloride (EDC) and N-Hydroxysuccinimide (NHS). The capture antibody, anti-human IgG (Fc), was diluted to 25 µg/mL with 10 mM sodium acetate (pH 5.0), and then injected at a flow rate of 10 µL/min to achieve a coupling amount of about 10,000 response units (RU). After injection of the capture antibody, 1 M ethanolamine was injected to block unreacted groups. For the kinetic measurements, the anti-TL1A monoclonal antibodies were diluted to 1 µg/mL and injected at 10 µL/min to ensure capture of about 200 RU by the anti-human Fc antibody. His-fib-hTL1A, His-fib-mTL1A, and His-fib-mfTL1A were each serially diluted to concentrations of, for example, 1.3 nM, 3.7 nM, 11.1 nM, 33.3 nM, and 100 nM, and injected at a flow rate of 30 µL/min from lowest to highest concentrations, with an association time of 90 seconds and a dissociation time of 1200 seconds. 3 M of MgCl₂ solution was injected at a flow rate of 10 µL/min for 30 seconds to regenerate the chip surface. The association rate (Kₐ) and the dissociation rate (K_{d}) were calculated by fitting the association and dissociation sensorgrams to a 1: 1 binding model using Biacore T200 Evaluation Software (version 3.2.1). The dissociation equilibrium constant (K_{D}) was calculated as the K_{d}/Kₐ ratio. The fitting results are shown in Tables 1, 2, and 3.

**Table 1. Affinity Constants of Anti-TL1A Monoclonal Antibodies Binding to Human TL1A (His-fib-hTL1A)**

| Antibodies | Kₐ(M⁻¹s⁻¹)) | K_{d}(s⁻¹) | K_{D}(M) |
|---|---|---|---|
| RVT-3101 | 6.521E+5 | 1.001E-4 | 1.536E-10 |
| PRA023 | 4.755E+5 | 1.686E-4 | 3.546E-10 |
| S4D3 | 5.323E+5 | 1.313E-4 | 2.467E-10 |
| S9H9 | 1.340E+7 | 5.012E-4 | 3.742E-11 |
| S7B11 | 4.704E+5 | 1.101E-4 | 2.341E-10 |

**Table 2. Affinity Constants of Anti-TL1A Monoclonal Antibodies Binding to Mouse TL1A (His-fib-mTL1A)**

| Antibodies | Kₐ(M⁻¹s⁻¹) | K_{d}(s⁻¹) | K_{D}(M) |
|---|---|---|---|
| RVT-3101 | 9.039E+5 | 4.358E-5 | 4.821E-11 |
| PRA023 | NB | / | / |
| S4D3 | NB | / | / |
| S9H9 | 6.857E+4 | 2.551E-3 | 3.719E-8 |
| S7B11 | 6.832E+4 | 3.657E-3 | 5.353E-8 |

| | | | |
|---|---|---|---|
| NB: No binding detected | | | |

**Table 3. Affinity Constants of Anti-TL1A Monoclonal Antibodies Binding to Monkey TL1A (His-fib-mfTL1A)**

| Antibodies | Kₐ(M⁻¹s⁻¹) | K_{d}(s⁻¹) | K_{D}(M) |
|---|---|---|---|
| RVT-3101 | 1.643E+6 | 4.515E-5 | 2.748E-11 |
| PRA023 | 1.124E+6 | 9.014E-5 | 8.023E-11 |
| S4D3 | 1.290E+6 | 6.309E-5 | 4.893E-11 |
| S9H9 | 2.900E+6 | 1.749E-4 | 6.031E-11 |
| S7B11 | 1.322E+6 | 6.930E-5 | 5.202E-11 |

### 3.2 Mouse Anti-TL1A Monoclonal Antibody Inhibits Caspase Activation in TL1A-stimulated TF-1 Cells

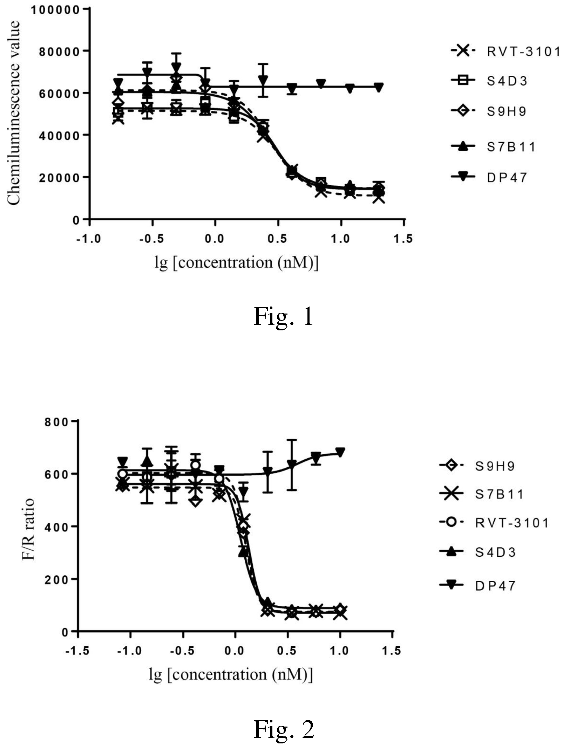

TF-1 cells (ATCC, CRL-2003, human erythroid leukemia cells) were purchased from ATCC, and their growth is completely dependent on IL-3 or GM-CSF and unresponsive to IL-5. The cells express the TL1A receptor DR3, and TL1A can stimulate caspase-3/7 activation in the cells. Cycloheximide (CHX) is an inhibitor of eukaryotic protein synthesis and can inhibit cellular protein synthesis. RPMI 1640 supplemented with 5% inactivated FBS was used as the test medium. TF-1 cells were resuspended in the test medium containing 2 µM cycloheximide to 2×10⁵ cells/mL and plated in a 96-well plate at 50 µL/well. The test antibodies were diluted with the test medium containing 800 ng/mL His-fib-hTL1A at a starting concentration of 40 nM (with 1.7-fold serial dilutions, for a total of 10 concentrations). Each dilution was added to TF-1 cells at 50 µL/well to a total volume of 100 µL/well and incubated at 37 °C and 5% CO₂ for 5 hours. Caspase-3/7 activity was detected using Caspase-glo 3/7 Kit (Promega, CAT#G8093), and full wavelength scanning was performed using a multifunctional microplate reader (Molecular Devices, SpectraMax^{®}I3X). Fitting analysis was performed using the chemiluminescence reading results. The results (FIG. 1 and Table 4) demonstrate that the mouse anti-TL1A monoclonal antibodies S4D3, S9H9 and S7B11 can effectively inhibit His-fib-hTL1A-induced caspase activation, and their activity is comparable to that of RVT-3101.

**Table 4. Results of Inhibitory Effect of Mouse Anti-TL1A Monoclonal Antibodies on Caspase Activation in TL1A-stimulated TF-1 Cells**

| Antibodies | IC₅₀(nM) |
|---|---|
| RVT-3101 | 3.129 |
| S4D3 | 3.047 |
| S9H9 | 2.686 |
| S7B11 | 2.686 |

### 3.3 Mouse Anti-TL1A Monoclonal Antibodies Inhibit NF-κB Activation in TL1A-stimulated 293T-hDR3-NF-κB-RE-luci Cells

The 293T-NF-κB-RE-luci cells, an NF-κB reporter gene cell line, was purchased from Inovogen Tech. Co. Ltd. (CAT# CS025). A 293T-hDR3-NF-κB-RE-luci cell line stably expressing hDR3 for evaluating the activity of anti-TL1A antibodies was obtained by transfecting the hDR3 gene into the cells via liposomes. DMEM supplemented with 2%FBS was used as the test medium. Test antibodies were diluted with the test medium containing 240 ng/mL His-fib-hTL1A at a starting concentration of 20 nM (with 1.7-fold serial dilutions, for a total of 10 concentrations). The 293T-hDR3-NF-κB-RE-luci cells were diluted to 4×10⁶ cells/mL with the test medium, and 50 µL of the cells were mixed with 50 µL of the antibody dilution, and then inoculated into a 96-well plate and cultured at 37 °C and 5% CO₂ for 20 hours. The activity of firefly luciferase and renilla luciferase was sequentially detected using Dual-glo Luciferase Detection Kit (Promega, E2920). Full wavelength scanning was performed using a multifunctional microplate reader (Molecular Devices, SpectraMax^{®}I3X), and the ratio of the firefly luciferase luminescence signal to the renilla luciferase luminescence signal was used for fitting analysis. The results (FIG. 2 and Table 5) demonstrate that the mouse anti-TL1A monoclonal antibodies S4D3, S9H9 and S7B11 can effectively inhibit NF-κB signaling induced by His-fib-hTL1A, with activity comparable to that of RVT-3101.

**Table 5. Results of Inhibitory Effect of Mouse Anti-TL1A Monoclonal Antibodies on NF-κB Activation in TL1A-stimulated 293T-hDR3-NF-κB-RE-luci Cells**

| Antibodies | IC₅₀ (nM) |
|---|---|
| S9H9 | 1.284 |
| S7B11 | 1.346 |
| RVT-3101 | 1.278 |
| S4D3 | 1.128 |

### Example 4: Screening of Fully-humanized Fab Recombinant Library

The fully-humanized Fab phage library was screened by a solid phase screening strategy (for the experimental protocol, see Phage Display: A Practical Approach, Clackson, T. (USA) and Lowman, H. B. (USA) (ed.), translated by Lan Ma et al., Chemical Industry Press, May 2008) using the recombinant His-fib-hTL1A and His-fib-mfTL1A prepared in Example 1. Three rounds of screening were performed by means of binding, elution, neutralization, infection and amplification. Finally, a monoclonal antibody H3F1+L28E1 (the amino acid sequence of H3F1VH is set forth in SEQ ID NO: 26; the amino acid sequence of L28E1V κ is set forth in SEQ ID NO: 30) that specifically binds to human, *Macaca fascicularis,* mouse or rat TL1As was obtained.

H3F1+L28E1 was prepared as an IgG1m3 allotype intact antibody using conventional molecular biological methods.

### Example 5: Identification of Fully-humanized Anti-TL1A Monoclonal Antibody

### 5.1 Affinity Analysis of Anti-TL1A Monoclonal Antibody H3F1+L28E1

Following the method described in Section 3.1, the affinity of the TL1A monoclonal antibody H3F1+L28E1 binding to TL1A from different species was analyzed using Biacore T200, and the results are shown in Tables 6, 7, 8, and 9.

**Table 6. Affinity Constants of Anti-TL1A Monoclonal Antibody H3F1+L28E1 Binding to Human TL1A (His-fib-hTL1A)**

| Antibodies | Kₐ(M⁻¹s⁻¹) | K_{d}(s^{- 1}) | K_{D}(M) |
|---|---|---|---|
| RVT-3101 | 7.063E+5 | 1.016E-4 | 1.438E-10 |
| PRA023 | 4.755E+5 | 1.686E-4 | 3.546E-10 |
| H3F1+L28E1 | 5.381E+6 | 1.562E-4 | 2.093E-11 |

**Table 7. Affinity Constants of Anti-TL1A Monoclonal Antibody H3F1+L28E1 Binding to Mouse TL1A (His-fib-mTL1A)**

| Antibodies | Kₐ(M⁻¹s⁻¹) | K_{d}(s⁻¹) | K_{D}(M) |
|---|---|---|---|
| RVT-3101 | 7.635E+5 | 5.92E-5 | 7.753E-11 |
| PRA023 | NB | / | / |
| H3F1+L28E1 | 9.643E+5 | 1.831E-3 | 1.898E-9 |

| | | | |
|---|---|---|---|
| NB: No binding detected | | | |

**Table 8. Affinity Constants of Anti-TL1A Monoclonal Antibody H3F1+L28E1 Binding to Rat TL1A (His-fib-rTL1A)**

| Antibodies | Kₐ(M⁻¹s⁻¹ ) | K_{d}(s⁻¹) | K_{D} (M) |
|---|---|---|---|
| RVT-3101 | 9.778E+5 | 4.243E-4 | 4.34E-10 |
| PRA023 | NB | / | / |
| H3F1+L28E1 | 8.037E+6 | 1.170E-3 | 1.456E-10 |

| | | | |
|---|---|---|---|
| NB: No binding detected | | | |

**Table 9. Affinity Constants of Anti-TL1A Monoclonal Antibody H3F1+L28E1 Binding to Monkey TL1A (His-fib-mfTL1A)**

| Antibodies | Kₐ(M⁻¹s⁻¹) | K_{d}(s⁻¹) | K_{D}(M) |
|---|---|---|---|
| RVT-3101 | 1.340E+6 | 6.700E-5 | 4.998E-11 |
| PRA023 | 1.124E+6 | 9.014E-5 | 8.023E-11 |
| H3F1+L28E1 | 4.191E+6 | 1.330E-4 | 3.173E-11 |

### 5.2 H3F1+L28E1 Inhibits Caspase Activation in TL1A-stimulated TF-1 Cells

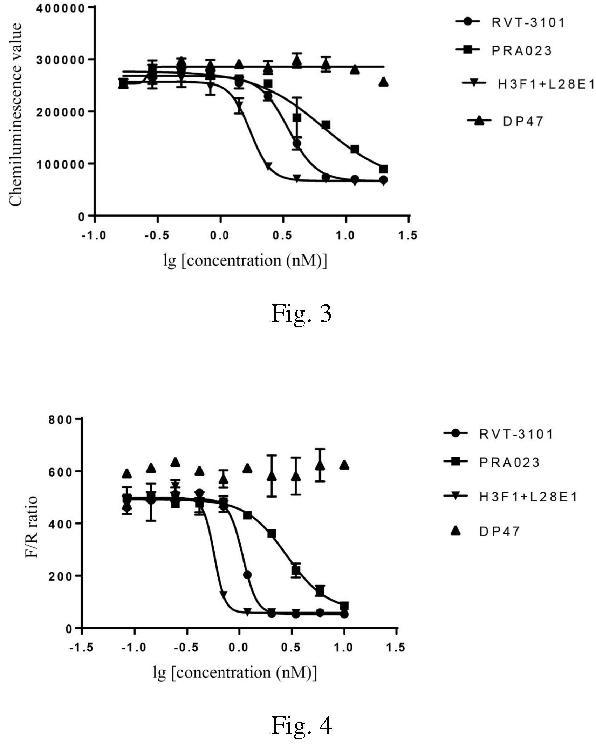

Following the method described in Section 3.2, the inhibitory effect of H3F1+L28E1 on caspase activation in TL1A-stimulated TF-1 cells was evaluated. Test antibodies were diluted with test medium containing 800 ng/mL His-fib-hTL1A at a starting concentration of 40 nM (with 1.7-fold serial dilutions, for a total of 10 concentrations). The results (FIG. 3 and Table 10) demonstrate that H3F1+L28E1 can effectively inhibit His-fib-hTL1A-induced caspase signaling, with activity superior to that of the control antibodies RVT-3101 and PRA023.

**Table 10. Inhibitory Effect of H3F1+L2831 on Caspase Activation in TL1A-stimulated TF-1 Cells**

| Antibodies | IC₅₀(nM) |
|---|---|
| RVT-3101 | 3.464 |
| PRA023 | 6.523 |
| H3F1+L28E1 | 1.734 |

### 5.3 H3F1+L28E1 Inhibits NF-κB Activation in TL1A-stimulated 293T-hDR3-NF-κB-RE-luci Cells

Following the method described in Section 3.3, the inhibitory effect of H3F1+L28E1 on NF-κB activation in TL1A-stimulated 293T-hDR3-NF-κB-RE-luci cells was evaluated. Test antibodies were diluted with the test medium containing 240 ng/mL His-fib-hTL1A at a starting concentration of 20 nM (with 1.7-fold serial dilutions, for a total of 10 concentrations). The results (FIG. 4 and Table 11) demonstrate that H3F1+L28E1 can effectively inhibit His-fib-hTL1A-stimulated NF-κB signaling, with activity superior to that of RVT-3101 and PRA023.

**Table 11. Inhibitory Effect of H3F1+L28E1 on NF-κB Activation in TL1A-stimulated 293T-hDR3-NF-κB-RE-luci Cells**

| Antibodies | IC₅₀(nM) |
|---|---|
| RVT-3101 | 1.082 |
| PRA023 | 2.813 |
| H3F1+L28E1 | 0.574 |

### 5.4 H3F1+L28E1 Inhibits the Ability of TL1A-stimulated PBMCs to Secrete Interferon-γ

NK cells or activated T cells express the TL1A receptor DR3 on the surface. With the help of co-stimulators such as IL-12/IL-18, TL1A can activate the downstream signaling pathway after binding to NK cells or activated T cells, ultimately resulting in the secretion of interferon-γ. The inhibitory effect of anti-TL1A antibodies on TL1A can be evaluated by detecting the content of interferon-γ in the cell supernatant.

Blood (50 mL) was collected from healthy volunteers who had signed informed consent. The inclusion criteria for volunteers were:
1. Age: ≥ 18 years;
2. No HIV and HBV infection;
3. Normal results of the blood routine test; and
4. Non-pregnant or non-lactating females.

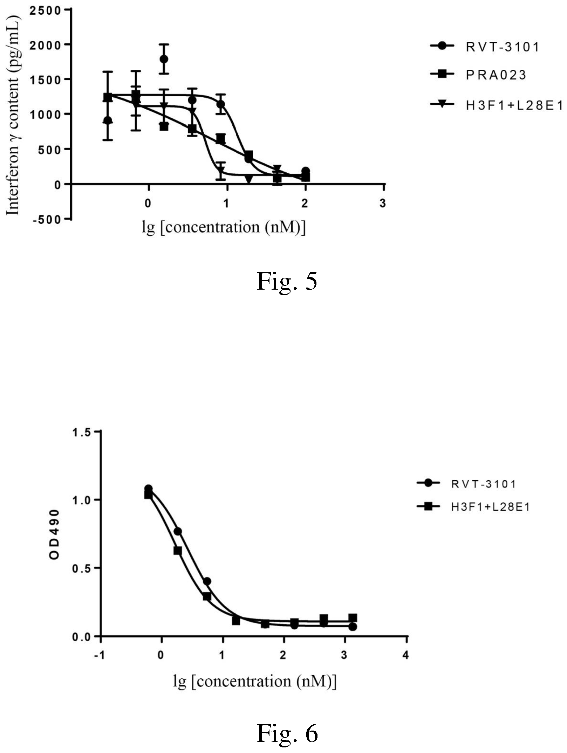

Human peripheral blood mononuclear cells (PBMCs) were isolated from the healthy volunteers' peripheral blood using Ficoll density gradient centrifugation. RPMI 1640 supplemented with 5% inactivated FBS was used as the test medium. PBMCs were resuspended to 6×10⁵ cells/mL in the test medium containing 0.5 ng/mL of IL-12 and 2 ng/mL of IL-18, plated in a 96-well plate at 100µL/wells, and allowed to stand for 3 hours at 37 °C and 5% CO₂. After 3 hours, test antibodies were diluted with the test medium containing 2.4 µg/mL His-fib-hTL1A, at a starting concentration of 200 nM (with 2.3-fold serial dilutions, for a total of 8 concentrations). The dilutions were added to the PBMCs at 100 µL/well with a total volume of 200 µL/well and incubated at 37 °C and 5% CO₂ for 20 hours. The interferon-γ content in the cell supernatant was measured with Human IFN-γ precoated ELISA Kit (Dayou, CAT#1110002). Absorbance was read at OD 450 nm using a microplate reader (800TS, Biotek). Standard curves of concentration and absorbance were plotted using the interferon-γ standard sample contained in the kit, and the interferon-γ content in the supernatant was obtained by conversion and analyzed by fitting. The results (FIG. 5 and Table 12) demonstrate that H3F1+L28E1 can effectively inhibit interferon-γ secretion from His-fib-hTL1A-stimulated PBMCs, with activity superior to that of RVT-3101 and PRA023.

**Table 12. Inhibitory Effect of H3F1+L28E1 on Interferon-γ Secretion by TL1A-stimulated PBMCs**

| Antibodies | IC₅₀(nM) |
|---|---|
| RVT-3101 | 13.61 |
| PRA023 | 6.545 |
| H3F1+L28E1 | 5.232 |

### Example 6: Anti-TL1A Monoclonal Antibody Selectively Neutralizes Functional Receptor DR3

Human DR3 (Recombinant Human DR3/TNFRSF25 Fc Chimera Protein, CF, R&D SYSTEMS, 943-D3-050) was biotinylated using Biotinylation Kit (EZ-Link^{™} Sulfo-NHS-Biotin, No-Weigh^{™} Format, Thermo Fisher, A39256). His-fib-hTL1A was coated on a 96-well ELISA plate at 4 µg/mL, 100 µL/well, and incubated overnight at 4°C. A blocking solution (3% skim milk-PBST) was used for blocking at 37°C for 1 hour. Anti-human TL1A monoclonal antibodies (RVT-3101 and H3F1+L28E1) were serially diluted using 10 µg/mL of biotinylated human DR3 at a starting concentration of 200 µg/mL (with 3-fold serial dilutions, for a total of 8 concentrations), and added to the blocked 96-well ELISA plate at 100 µL/well and incubated at 37°C for 1 hour. The ELISA plate was washed with PBST, and then horseradish peroxidase-labeled Streptavidin/HRP (Bioss, bs-0437P-HRP) was added and incubated at 37 °C for 1 hour. The ELISA plate was washed with PBST. OPD substrate chromogenic solution was added, and then terminated with 1 M H₂SO₄ after 5-10 minutes. Single wavelength optical density values at 490 nm were measured using a microplate reader. The results of the ELISA assay are shown in FIG. 6: RVT-3101 and H3F1+L28E1 can prevent human DR3 from binding to human TL1A.

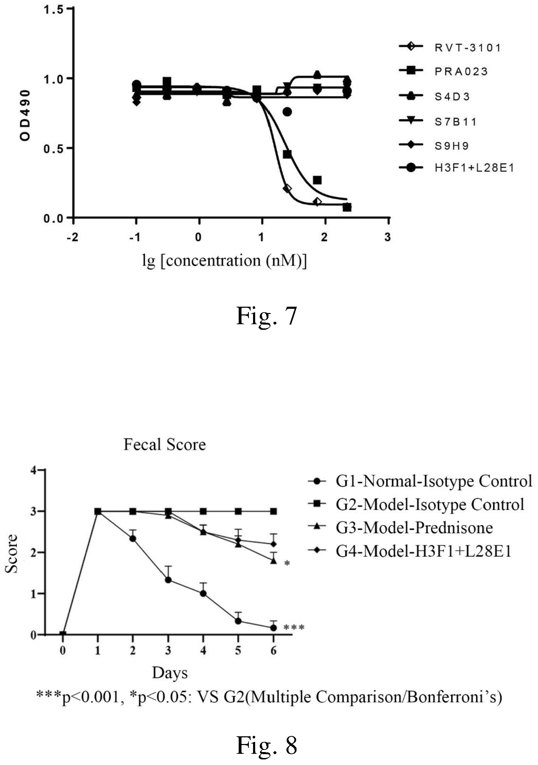

Human DcR3 (Recombinant Human DcR3/TNFRSF6B Fc Chimera Protein, CF, R&D SYSTEMS, 142-DC-100) was coated on a 96-well ELISA plate at 3 µg/mL, 100 µL/well, and incubated overnight at 4°C. A blocking solution (3% skim milk-PBST) was used for blocking at 37 °C for 1 hour. Anti-human TL1A monoclonal antibodies (RVT-3101, PRA023, S4D3, S9H9, S7B11 and H3F1+L28E1) were each serially diluted using 3 µg/mL of His-fib-hTL1A at a starting concentration of 100 µg/mL (with 3-fold serial dilutions, for a total of 11 concentrations). The dilutions were added to the blocked 96-well ELISA plate at 100 µL/well and incubated at 37°C for 1 hour. The ELISA plate was washed with PBST followed by the addition of HRP-labeled anti-His-tag mouse monoclonal antibody (Cwbio, cw0285M) and incubated at 37°C for 1 hour. The ELISA plate was washed with PBST. OPD substrate chromogenic solution was added, and terminated with 1 M H₂SO₄ after 5-10 minutes. Single wavelength optical density values at 490 nm were measured using a microplate reader. The ELISA analysis results are shown in FIG. 7: RVT-3101 and PRA023 can prevent human DcR3 from binding to human TL1A, and S4D3, S9H9, S7B11 and H3F1+L28E1 do not prevent human DcR3 from binding to human TL1A.

As the common decoy receptor for TNF family cytokines (Fas-L, LIGHT, and TL1A), DcR3 has a natural antagonistic function *in vivo*. S4D3, S9H9, S7B11 and H3F1+L28E1 are capable of neutralizing DR3 rather than DcR3, indicating that these antibodies do not disrupt the natural antagonistic activity of DcR3, which helps maintain the homeostatic balance of DcR3 *in vivo* and exhibits better safety.

### Example 7: Effect of Anti-TL1A Monoclonal Antibody on DNBS-induced Acute Ulcerative Colitis in Rats

Male Wistar rats without a history of drug administration were purchased from Shanghai SLAC Laboratory Animal Co., Ltd., and adapted to the animal room environment 3 days in advance. Before the DNBS modelling, the rats were fasted for 40 hours and subcutaneously injected with 5% glucose injection (10 mL/kg) as an energy supplement during the fasting period. The DNBS powder was dissolved in 30% ethanol at a final concentration of 60 mg/mL. The fasted rats were anesthetized on day 0 with Zoletil (i.p., 25-50 mg/kg) and Xylazine (i.p., 5-10 mg/kg). The animal grouping and dosing regimens are shown in Table 13. Among these, rat colitis was induced using DNBS enema by extending a catheter into the colon from the anus for Groups G2 to G4. Enema was performed for Group G1 using 30% ethanol in the same manner. The isotype control was DP47 antibody.

**Table 13. Grouping and Dosing Regimens**

| Groups | no. | Concentration (mg/mL) | Dosage# | | Route | Regimen |
|---|---|---|---|---|---|---|
| | | | mL/kg | mg/kg | | |
| G1-Normal-Iso type Control | 6 | 1 | 10 | 10 | i.p. | Day 0 and Day 3 |
| G2-Model-Isotype Control | 6 | 1 | 10 | 10 | i.p. | Day 0 and Day 3 |
| G3-Model-Prednisone | 10 | 0.9 | 10 | 9 | p.o. | Once daily, from day 0 to day 6 |
| G4-Model-H3F 1+L28E1 | 10 | 1 | 10 | 10 | i.p. | Day 0 and Day 3 |

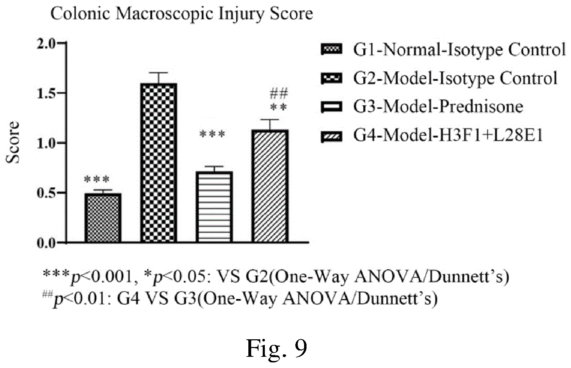

The experimental animals' fecal consistency was scored daily during the study period (0 = normal, 1 = wet/viscous, 2 = soft, and 3 = liquid). Body weights of the experimental animals were measured and recorded daily. On day 6, all animals were euthanized with excess carbon dioxide, the abdominal cavity was opened, and the colon was removed, longitudinally dissected, and observed for ulceration after rinsing. The length, weight, and ulcer area of the colon were recorded and scored for colon macroscopic damage. The results (FIG. 8 and FIG. 9) demonstrate that the fecal consistency and intestinal damage of the animals in Group G4 (H3F1+L28E1) were significantly improved compared to those in Group G2 (Model-Isotype Control).

All patents, patent application publications, and non-patent literatures mentioned and/or listed in the present application are incorporated herein by reference in their entirety. Exemplary embodiments of the inventions of the present invention are described above, however, those skilled in the art will be able to modify or improve the exemplary embodiments described herein without departing from the spirit and scope of the present application, and variations or equivalents derived therefrom are also within the scope of the present application.

### Sequence Listing

**SEQ ID NO: 1**
   KYDIN
**SEQ ID NO: 2**
   WIFPGDGRTDYNEKFKG
**SEQ ID NO: 3**
   YGYALDY
**SEQ ID NO: 4**
   RSSQNIVHSNGDTYLE
**SEQ ID NO: 5**
   KVSNRFS
**SEQ ID NO: 6**
   FQGSHFPYT
**SEQ ID NO: 7**
   NYWLG
**SEQ ID NO: 8**
   DIHPGRGNIFYNEKFKG
**SEQ ID NO: 9**
   GYDTFDY
**SEQ ID NO: 10**
   KASQDVSTAVA
**SEQ ID NO: 11**
   SASYRYT
**SEQ ID NO: 12**
   QQHYSTPWT
**SEQ ID NO: 13**
   DIHPGRGNIYYNEKFKG
**SEQ ID NO: 14**
   RASKSISKYLA
**SEQ ID NO: 15**
   SGSTLQS
**SEQ ID NO: 16**
   QQHNEYPYT
**SEQ ID NO: 17**
   SYDIN
**SEQ ID NO: 18**
   WLNPNSGNTGYAQKFQG
**SEQ ID NO: 19**
   EIPESAAIEY
**SEQ ID NO: 20**
   TSSSSDIGAGLGVH
**SEQ ID NO: 21**
   GYYNRPS
**SEQ ID NO: 22**
   QSYDASLTGI
**SEQ ID NO: 23** 
QVQLKESGSDLATPGASVKLSCKVSGYTFTKYDINWVRQRPEQGLEWIGWIFPGDGRTDY
NEKFKGKATLTIDTSSSTAYMQLSRLTSEDSAVYFCARYGYALDYWGQGTSVTVSS
**SEQ ID NO: 24** 
QVQLKQSGAELVRPGTSVKISCKASGYAFTNYWLGWIKQRPGHGLEWIGDIHPGRGNIFY
NEKFKGKATLTADKSSSTAYMQLSSLTSEDSAVYFCAEGYDTFDYWGQGTTLTVSA
**SEQ ID NO: 25** 
QVKLQQSGGELVRPGTSVKISCKASGYAFTNYWLGWIKQRPGHGLEWIGDIHPGRGNIYY
NEKFKGKATLTADKSSSTAYMQLSSLTSEDSAVYFCAEGYDTFDYWGQGTTLTVSS
**SEQ ID NO: 26** 
QVQLVQSGAEVKKPGASVKVSCKASGYTFTSYDINWVRQAPGQGLEWMGWLNPNSGNT
GYAQKFQGRVTMTADRSTSTAYMELSSLRSEDTAVYYCAREIPESAAIEYWGQGTLVTVSS
**SEQ ID NO: 27** 
DVVMTQTPLSLPVSLGDQASISCRSSQNIVHSNGDTYLEWFLQKPGQSPKLLIYKVSNRFS
GVPDRFSGSGSGTDFTLKISRVEAEDLGVYYCFQGSHFPYTFGGGTKLEMK
**SEQ ID NO: 28** 
DIVMTQSHKFMSTSVGDRVSITCKASQDVSTAVAWYQQKPGQSPKLLIYSASYRYTGVPD
RFTGSGSGTDFTFTISSVQAEDLAVYYCQQHYSTPWTFGGGTKLEMK
**SEQ ID NO: 29** 
DIVMTQSPSYLAASPGETITINCRASKSISKYLAWYQEKPGKTNKLLIYSGSTLQSGIPSRFS
GSGSGTDFTLTISSLEPEDFAMYYCQQHNEYPYTFGGGTKLEIK
**SEQ ID NO: 30** 
QSVLTQPPSVSGAPGQRVTISCTSSSSDIGAGLGVHWYQQLPGTAPKLLIEGYYNRPSGVP
DRFSGSKSGTSASLTITGLLPEDEGDYYCQSYDASLTGIFGGGTKLTVL
**SEQ ID NO: 31** 
LKGQEFAPSHQQVYAPLRADGDKPRAHLTVVRQTPTQHFKNQFPALHWEHELGLAFTKN
RMNYTNKFLLIPESGDYFIYSQVTFRGMTSECSEIRQAGRPNKPDSITVVITKVTDSYPEPT
QLLMGTKSVCEVGSNWFQPIYLGAMFSLQEGDKLMVNVSDISLVDYTKEDKTFFGAFLL
**SEQ ID NO: 32** 
LKGQEFAPSHQQVYAPLRADGDKPRAHLTVVRQTPTQHLKNQFPALHWEHELGLAFTKN
RMNYTNKFLLIPESGDYFVYSQVTFRGMTSECSEIRQAGRPNKPDSITVVITKVTDSYPEPT
QLLMGTKSVCEVGSNWFQPIYLGAMFSLQEGDKLMVNVSDISLVDYTKEDKTFFGAFLL
**SEQ ID NO: 33** 
ITEERSEPSPQQVYSPPRGKPRAHLTIKKQTPAPHLKNQLSALHWEHDLGMAFTKNGMKY
INKSLVIPESGDYFIYSQITFRGTTSVCGDISRGRRPNKPDSITMVITKVADSYPEPARLLTGS
KSVCEISNNWFQSLYLGATFSLEEGDRLMVNVSDISLVDYTKEDKTFFGAFLL
**SEQ ID NO: 34** 
VTEERSAPSAQPVYTPSRDKPKAHLTIMRQTPVPHLKNELAALHWENNLGMAFTKNRMN
YTNKFLVIPESGDYFIYSQITFRGTTSECGDISRVRRPKKPDSITVVITKVADSYPEPAHLLTG
TKSVCEISSNWFQPIYLGAMFSLEEGDRLMVNVSDISLVDYTKEDKTFFGAFLI
**SEQ ID NO: 35**
   GYIPEAPRDGQAYVRKDGEWVLLSTFL
**SEQ ID NO: 36**
   HHHHHH
**SEQ ID NO: 37** 
ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKRVEPKSCDKTHTCPPCPAPELLGGPSV
FLFPPKPKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSREEMT
KNQVSLTCLVKGFYPSDIAVEWESNGQPENNYKTTPPVLDSDGSFFLYSKLTVDKSRWQQ
GNVFSCSVMHEALHNHYTQKSLSLSPGK
**SEQ ID NO: 38** 
ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKRVEPKSCDKTHTCPPCPAPEFEGGPSV
FLFPPKPKDTLMISRTPEVTCVVVDVSHEDPEVKFNWYVDGVEVHNAKTKPREEQYNST
YRVVSVLTVLHQDWLNGKEYKCKVSNKALPASIEKTISKAKGQPREPQVYTLPPSREEMT
KNQVSLTCLVKGFYPSDIAVEWESNGQPENNYKTTPPVLDSDGSFFLYSKLTVDKSRWQQ
GNVFSCSVMHEALHNHYTQKSLSLSPGK
**SEQ ID NO: 39** 
ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKRVEPKSCDKTHTCPPCPAPEFEGGPSV
FLFPPKPKDTLYITREPEVTCVVVDVSHEDPEVKFNWYVDGVEVHNAKTKPREEQYNSTY
RVVSVLTVLHQDWLNGKEYKCKVSNKALPASIEKTISKAKGQPREPQVYTLPPSREEMTK
NQVSLTCLVKGFYPSDIAVEWESNGQPENNYKTTPPVLDSDGSFFLYSKLTVDKSRWQQG
NVFSCSVMHEALHNHYTQKSLSLSPGK
**SEQ ID NO: 40**
**SEQ ID NO: 41** 
GQPKAAPSVTLFPPSSEELQANKATLVCLISDFYPGAVTVAWKADSSPVKAGVETTTPSKQ
SNNKYAASSYLSLTPEQWKSHRSYSCQVTHEGSTVEKTVAPTECS
**SEQ ID NO: 42** 
HHHHHHGGGGSGYIPEAPRDGQAYVRKDGEWVLLSTFLGGGGSLKGQEFAPSHQQVYAP
LRADGDKPRAHLTVVRQTPTQHFKNQFPALHWEHELGLAFTKNRMNYTNKFLLIPESGD
YFIYSQVTFRGMTSECSEIRQAGRPNKPDSITVVITKVTDSYPEPTQLLMGTKSVCEVGSN
WFQPIYLGAMFSLQEGDKLMVNVSDISLVDYTKEDKTFFGAFLL
**SEQ ID NO: 43** 
HHHHHHGGGGSGYIPEAPRDGQAYVRKDGEWVLLSTFLGGGGSLKGQEFAPSHQQVYAP
LRADGDKPRAHLTVVRQTPTQHLKNQFPALHWEHELGLAFTKNRMNYTNKFLLIPESGD
YFVYSQVTFRGMTSECSEIRQAGRPNKPDSITVVITKVTDSYPEPTQLLMGTKSVCEVGSN
WFQPIYLGAMFSLQEGDKLMVNVSDISLVDYTKEDKTFFGAFLL
**SEQ ID NO: 44** 
HHHHHHGGGGSGYIPEAPRDGQAYVRKDGEWVLLSTFLGGGGSITEERSEPSPQQVYSPP
RGKPRAHLTIKKQTPAPHLKNQLSALHWEHDLGMAFTKNGMKYINKSLVIPESGDYFIYS
QITFRGTTSVCGDISRGRRPNKPDSITMVITKVADSYPEPARLLTGSKSVCEISNNWFQSLYL
GATFSLEEGDRLMVNVSDISLVDYTKEDKTFFGAFLL
**SEQ ID NO: 45** 
HHHHHHGGGGSGYIPEAPRDGQAYVRKDGEWVLLSTFLGGGGSVTEERSAPSAQPVYTP
SRDKPKAHLTIMRQTPVPHLKNELAALHWENNLGMAFTKNRMNYTNKFLVIPESGDYFI
 
YSQITFRGTTSECGDISRVRRPKKPDSITVVITKVADSYPEPAHLLTGTKSVCEISSNWFQPIY
LGAMFSLEEGDRLMVNVSDISLVDYTKEDKTFFGAFLI
**SEQ ID NO: 46**
   TGCATTTGAACTCCTTGCC
**SEQ ID NO: 47**
   CCATCAATCTTCCACTTGAC
**SEQ ID NO: 48** 
QVQLKESGSDLATPGASVKLSCKVSGYTFTKYDINWVRQRPEQGLEWIGWIFPGDGRTDY
NEKFKGKATLTIDTSSSTAYMQLSRLTSEDSAVYFCARYGYALDYWGQGTSVTVSSGGGG
SGGGGSGGGGSDVVMTQTPLSLPVSLGDQASISCRSSQNIVHSNGDTYLEWFLQKPGQSP
KLLIYKVSNRFSGVPDRFSGSGSGTDFTLKISRVEAEDLGVYYCFQGSHFPYTFGGGTKLE
MK
**SEQ ID NO: 49** 
QVQLKQSGAELVRPGTSVKISCKASGYAFTNYWLGWIKQRPGHGLEWIGDIHPGRGNIFY
NEKFKGKATLTADKSSSTAYMQLSSLTSEDSAVYFCAEGYDTFDYWGQGTTLTVSAGGGG
SGGGGSGGGGSDIVMTQSHKFMSTSVGDRVSITCKASQDVSTAVAWYQQKPGQSPKLLIY
SASYRYTGVPDRFTGSGSGTDFTFTISSVQAEDLAVYYCQQHYSTPWTFGGGTKLEMK
**SEQ ID NO: 50** 
QVKLQQSGGELVRPGTSVKISCKASGYAFTNYWLGWIKQRPGHGLEWIGDIHPGRGNIYY
NEKFKGKATLTADKSSSTAYMQLSSLTSEDSAVYFCAEGYDTFDYWGQGTTLTVSSGGGG
SGGGGSGGGGSDIVMTQSPSYLAASPGETITINCRASKSISKYLAWYQEKPGKTNKLLIYS
GSTLQSGIPSRFSGSGSGTDFTLTISSLEPEDFAMYYCQQHNEYPYTFGGGTKLEIK
**SEQ ID NO: 51** 
QVQLVQSGAEVKKPGASVKVSCKASGYDFTYYGISWVRQAPGQGLEWMGWISTYNGNT
HYARMLQGRVTMTTDTSTRTAYMELRSLRSDDTAVYYCARENYYGSGAYRGGMDVWG
QGTTVTVSS
**SEQ ID NO: 52** 
EIVLTQSPATLSLSPGERATLSCRASQSVSSYLAWYQQKPGQAPRLLIYDASNRATGIPARFS
GSGSGTDFTLTISSLEPEDFAVYYCQQRSNWPWTFGQGTKVEIK
**SEQ ID NO: 53** 
QVQLVQSGAEVKKPGASVKVSCKASGFDIQDTYMHWVKQRPGQGLEWMGRIDPASGHT
KYDPKFQVRVTITRDTSTSTVYLELSSLRSEDTAVYYCARSGGLPDVWGQGTTVTVSS
**SEQ ID NO: 54** 
EIVLTQSPGTLSLSPGERATLSCRASSSVSYMYWYQQKPGQAPRPLIYATSNLASGIPDRFS
GSGSGTDFTLTISRLEPEDFAVYYCQQWEGNPRTFGGGTKLEIK
**SEQ ID NO: 55** 
EVQLLESGGGLVQPGGSLRLSCAASGFTFSSYAMSWVRQAPGKGLEWVSAISGSGGSTYY
ADSVKGRFTISRDNSKNTLYLQMNSLRAEDTAVYYCAKGSGFDYWGQGTLVTVSS
**SEQ ID NO: 56**

EIVLTQSPGTLSLSPGERATLSCRASQSVSSSYLAWYQQKPGQAPRLLIYGASSRATGIPDRF
SGSGSGTDFTLTISRLEPEDFAVYYCQQYGSSPLTFGQGTKVEIK

### Reference

1. Xu WD, Li R, Huang AF. Role of TL1A in Inflammatory Autoimmune Diseases: A Comprehensive Review. Front Immunol. 2022 Jul 14;13: 891328.
2. Migone TS, Zhang J, Luo X, Zhuang L, Chen C, Hu B, Hong JS, Perry JW, Chen SF, Zhou JX, Cho YH, Ullrich S, Kanakaraj P, Carrell J, Boyd E, Olsen HS, Hu G, Pukac L, Liu D, Ni J, Kim S, Gentz R, Feng P, Moore PA, Ruben SM, Wei P. TL1A is a TNF-like ligand for DR3 and TR6/DcR3 and functions as a T cell costimulator. Immunity. 2002 Mar;16(3):479-92.
3. Valatas V, Kolios G, Bamias G. TL1A (TNFSF15) and DR3 (TNFRSF25): A Co-stimulatory System of Cytokines with Diverse Functions in Gut Mucosal Immunity. Front Immunol. 2019 Mar 27; 10:583.
4. Ramos GP, Papadakis KA. Mechanisms of Disease: Inflammatory Bowel Diseases. Mayo Clin Proc. 2019 Jan;94(1):155-165.
5. Li N, Ye M. Advances in biological agents in the treatment of inflammatory bowel disease[J]. Yixue Xinzhi Zazhi, 2022, 32(4): 310-320.
6. Krebber A, Bornhauser S, Burmester J, Honegger A, Willuda J, Bosshard HR, Plückthun A. Reliable cloning of functional antibody variable domains from hybridomas and spleen cell repertoires employing a reengineered phage display system. J Immunol Methods. 1997 Feb 14;201(1):35-55.
7. CN201510097117.0.
8. Phage Display: A Practical Approach, Clackson, T. (USA) and Lowman, H. B. (USA) (ed.), translated by Lan Ma et al., Chemical Industry Press, May 2008.5.
9. US20150132311A1
10. US20210122828A1
11. US20160200833A1

## Claims

1. An antibody that binds to tumor necrosis factor-like ligand 1A (TL1A) comprising a heavy chain variable region and a light chain variable region, wherein
the heavy chain variable region comprises the amino acid sequence of HCDR1, HCDR2 and HCDR3 as set forth in SEQ ID NO: 1, 2 and 3, and the light chain variable region comprises the amino acid sequence of LCDR1, LCDR2 and LCDR3 as set forth in SEQ ID NO: 4, 5 and 6; or the heavy chain variable region comprises the amino acid sequence of HCDR1, HCDR2 and HCDR3 as set forth in SEQ ID NO: 7, 8 and 9, and the light chain variable region comprises the amino acid sequence of LCDR1, LCDR2 and LCDR3 as set forth in SEQ ID NO: 10, 11 and 12; or
the heavy chain variable region comprises the amino acid sequence of HCDR1, HCDR2 and HCDR3 as set forth in SEQ ID NO: 7, 13, and 9, and the light chain variable region comprises the amino acid sequence of LCDR1, LCDR2 and LCDR3 as set forth in SEQ ID NO: 14, 15 and 16; or
the heavy chain variable region comprises the amino acid sequence of HCDR1, HCDR2 and HCDR3 as set forth in SEQ ID NO: 17, 18 and 19, and the light chain variable region comprises the amino acid sequence of LCDR1, LCDR2 and LCDR3 as set forth in SEQ ID NO: 20, 21 and 22;
wherein the amino acid sequences of HCDRs and LCDRs are defined according to Kabat numbering scheme.

2. The antibody of claim 1, wherein the heavy chain variable region of the antibody has the amino acid sequence as set forth in SEQ ID NO: 23, 24, 25 or 26.

3. The antibody of claim 1, wherein the light chain variable region of the antibody has the amino acid sequence as set forth in SEQ ID NO: 27, 28, 29 or 30.

4. The antibody of any one of claims 1 to 3, wherein
the heavy chain variable region of the antibody has the amino acid sequence as set forth in SEQ ID NO: 23, and the light chain variable region of the antibody has the amino acid sequence as set forth in SEQ ID NO: 27; or
the heavy chain variable region of the antibody has the amino acid sequence as set forth in SEQ ID NO: 24, and the light chain variable region of the antibody has the amino acid sequence as set forth in SEQ ID NO: 28; or
the heavy chain variable region of the antibody has the amino acid sequence as set forth in SEQ ID NO: 25, and the light chain variable region of the antibody has the amino acid sequence as set forth in SEQ ID NO: 29; or
the heavy chain variable region of the antibody has the amino acid sequence as set forth in SEQ ID NO: 26, and the light chain variable region of the antibody has the amino acid sequence as set forth in SEQ ID NO: 30.

5. The antibody of any one of claims 1 to 4, wherein the heavy chain variable region of the antibody has an amino acid sequence with at least 90% identity to SEQ ID NO: 23, 24, 25, or 26, and the light chain variable region of the antibody has an amino acid sequence with at least 90% identity to SEQ ID NO: 27, 28, 29, or 30.

6. The antibody of any one of claims 1 to 5, wherein
the antibody is an intact antibody, a Fab fragment, an F(ab')₂ fragment or a single-chain Fv fragment (scFv), preferably the antibody is a fully-humanized antibody; and/or
the antibody is a monoclonal antibody; and/or
the antibody further comprises a heavy chain constant region selected from the group consisting of an IgG1 subtype, an IgG2 subtype, and an IgG4 subtype; preferably, the heavy chain constant region is an IgG1 subtype; more preferably, the heavy chain constant region comprises an Fc fragment of the IgG1 subtype heavy chain constant region, and the Fc fragment comprises the amino acid F at position 234, the amino acid E at position 235, and the amino acid S at position 331, and/or the Fc fragment comprises the amino acid Y at position 252, the amino acid T at position 254, and the amino acid E at position 256; wherein the amino acid sequence of the antibody constant region is determined according to EU numbering scheme; and/or
the antibody further comprises a light chain constant region selected from the group consisting of a κ subtype and a λ subtype.

7. The antibody of any one of claims 1 to 6, wherein
the antibody binds to TL1A of a primate and/or a rodent; preferably, the primate is a human or a member of the Cercopithecidae (e.g., *Macaca fascicularis*), and/or the rodent is a member of the Muridae (e.g., rat or mouse); and/or
the antibody inhibits binding of TL1A to death receptor 3 (DR3); and/or
the antibody does not inhibit binding of TL1A to decoy receptor 3 (DcR3); and/or
the antibody inhibits caspase activation in TL1A-stimulated cells; and/or
the antibody inhibits NF-κB activation in TL1A-stimulated cells; and/or
the antibody inhibits the ability of TL1A-stimulated PBMCs to secrete interferon-γ.

8. A nucleic acid molecule encoding the antibody of any one of claims 1 to 7.

9. A pharmaceutical composition comprising the antibody of any one of claims 1 to 7 and a pharmaceutically acceptable excipient, diluent or carrier.

10. The pharmaceutical composition of claim 9 for use in the prevention or treatment of a TL1A-related disease; preferably, the TL1A-related disease is selected from the group consisting of rheumatoid arthritis, inflammatory bowel disease (e.g., Crohn's disease (CD) and ulcerative colitis (UC)), psoriasis, primary biliary cirrhosis, systemic lupus erythematosus, ankylosing spondylitis, and systemic sclerosis-related interstitial lung disease.

11. Use of the antibody of any one of claims 1 to 7, or the pharmaceutical composition of claim 9 or 10, for the manufacture of a medicament for the prevention or treatment of a TL1A-related disease; preferably, the TL1A-related disease is selected from the group consisting of rheumatoid arthritis, inflammatory bowel disease (e.g., Crohn's disease (CD) and ulcerative colitis (UC)), psoriasis, primary biliary cirrhosis, systemic lupus erythematosus, ankylosing spondylitis, and systemic sclerosis-related interstitial lung disease.

12. A method of preventing or treating a TL1A-related disease comprising administering to a subject in need thereof the antibody of any one of claims 1 to 7 or the pharmaceutical composition of claim 9 or 10; preferably, the TL1A-related disease is selected from the group consisting of rheumatoid arthritis, inflammatory bowel disease (e.g., Crohn's disease (CD) and ulcerative colitis (UC)), psoriasis, primary biliary cirrhosis, systemic lupus erythematosus, ankylosing spondylitis, and systemic sclerosis-related interstitial lung disease.
